# EUROPEAN PATENT APPLICATION

(11) **EP 4 725 522 A1**
(43) Date of publication of application: **15.04.2026**
(21) Application number: 24864986.5
(22) Date of filing: 15.05.2024
(51) Int. Cl.: A61M 5/168, A61M 5/142, A61M 5/145

(54) **PHARMACEUTICAL LIQUID ADMINISTRATION DEVICE AND ROTATION DETECTION DEVICE**

(30) Priority: 12.09.2023 JP 2023147459
(71) Applicant: Terumo Kabushiki Kaisha, Tokyo 151-0072 (JP)
(72) Inventor: KONDO, Akira, Ashigarakami-gun, Kanagawa 259-0151 (JP); HASEGAWA, Makoto, Ashigarakami-gun, Kanagawa 259-0151 (JP); YAKUSHIJI, Yusuke, Ashigarakami-gun, Kanagawa 259-0151 (JP)
(74) Representative: KIPA AB
(86) International application number: PCT/JP2024/017965
(87) International publication number: WO 2025/057478

(57) **Abstract**

A rotating body (a second gear (62)) of a medicinal solution administration device (10) has a to-be-irradiated portion (80) that is an outer surface along the axis (Ax) of the rotating body. A rotation sensor (70) includes a light emitting element (72) that emits an irradiation light beam toward the to-be-irradiated portion (80), and a light receiving element (74) that receives a reflected light beam from the to-be-irradiated portion (80) of the rotating body. The to-be-irradiated portion (80) includes at least two regions having different intensities of the reflected light beam in the circumferential direction of the rotating body. The intensity of the reflected light beam changes while the to-be-irradiated portion (80) makes one rotation about the axis. The control unit (58) detects the rotational state of the rotating body based on a change in the current value corresponding to the intensity of the reflected light beam.

## Description

### Technical Field

The present invention relates to a medicinal solution administration device and a rotation detection device.

### Background Art

Conventionally, detection of rotation of a rotating body in a medicinal solution administration device using an encoder is known as disclosed in JP 2013-537844 A, for example.

### Summary of Invention

In the medicinal solution administration device of JP 2013-537844 A, rotation of a motor is detected using an encoder attached to a motor shaft. In such a structure, the encoder protrudes significantly in a direction perpendicular to the axis of the motor shaft. Thus, it is difficult to reduce the size of the medicinal solution administration device.

It is therefore an object of the present invention to solve the above-described problem.

(1) A first aspect of the present invention is a medicinal solution administration device including a medicinal solution container that stores a medicinal solution, and has a medicinal solution discharge port at a distal end; a plunger that pushes out the medicinal solution in the medicinal solution container through the medicinal solution discharge port; a drive mechanism including a motor and a rotating body that transmits rotation of the motor to the plunger, the drive mechanism being adapted to move the plunger toward the distal end; a rotation sensor for detecting rotation of the rotating body; and a control unit, in which the rotating body has a to-be-irradiated portion that is an outer surface along an axis of the rotating body, the rotation sensor includes a light emitting element that emits an irradiation light beam toward the to-be-irradiated portion, and a light receiving element that receives a reflected light beam from the to-be-irradiated portion of the rotating body, the to-be-irradiated portion includes at least two regions having different intensities of the reflected light beam in a circumferential direction of the rotating body, the intensity of the reflected light beam changes while the to-be-irradiated portion makes one rotation around the axis, and the control unit detects a rotational state of the rotating body based on a change in a current value corresponding to the intensity of the reflected light beam.

In such a medicinal solution administration device, an irradiation light beam is emitted toward the to-be-irradiated portion, which is the outer surface along the axis of the rotating body, and a reflected light beam from the to-be-irradiated portion is received, and then, the rotational state of the rotating body is detected based on a change in the current value corresponding to the intensity of the reflected light beam. Thus, the size of the medicinal solution administration device can be easily reduced.

(2) In the medicinal solution administration device according to (1) above, an outer peripheral surface of the to-be-irradiated portion may include a first to-be-irradiated surface and a second to-be-irradiated surface having an intensity of the reflected light beam lower than that of the first to-be-irradiated surface.

According to such a configuration, at least two regions having different intensities of the reflected light beam can be easily provided.

(3) In the medicinal solution administration device according to (2) above, the to-be-irradiated portion may include a metal portion and a resin portion having an intensity of the reflected light beam lower than that of the metal portion, and the metal portion may be provided with the first to-be-irradiated surface, and the resin portion may be provided with the second to-be-irradiated surface.

According to such a configuration, the to-be-irradiated portion can be easily provided with two regions having different intensities of the reflected light beam.

(4) In the medicinal solution administration device according to (2) above, a cross-section of the to-be-irradiated portion perpendicular to the axis may have a circular shape, half of a circumference of an outer surface of the to-be-irradiated portion may correspond to the first to-be-irradiated surface, and another half of the circumference of the outer surface of the to-be-irradiated portion may correspond to the second to-be-irradiated surface.

According to such a configuration, a region where the intensity of the reflected light beam is high and a region where the intensity of the reflected light beam is low while the to-be-irradiated portion makes one rotation are made equal in size. Thus, the resolution of rotation detection can be enhanced.

(5) In the medicinal solution administration device according to (1) above, a cross-section of the to-be-irradiated portion perpendicular to the axis may have a circular shape, and the to-be-irradiated portion may be provided with a through-hole penetrating the to-be-irradiated portion in a radial direction of the circular shape.

According to such a configuration, at least two regions having different intensities of the reflected light beam can be easily provided.

(6) In the medicinal solution administration device according to (1) above, a cross-section of the to-be-irradiated portion perpendicular to the axis may have a polygonal shape.

According to such a configuration, it is also possible to detect the rotational state of the rotating body based on a change in the current value corresponding to the intensity of the reflected light beam.

(7) In the medicinal solution administration device according to any one of (1) to (6) above, a maximum value of the current value while the to-be-irradiated portion makes one rotation about the axis may be 10 times or more a minimum value of the current value.

Such a configuration enhances the accuracy of rotation detection.

(8) In the medicinal solution administration device according to any one of (1) to (7) above, the control unit may measure the number of rotations of the rotating body based on a change in the current value corresponding to the intensity of the reflected light beam.

Measuring the number of rotations can grasp whether the delivery of the medicinal solution is complete.

A second aspect of the present invention is a rotation detection device including a rotating body; and a rotation sensor that detects rotation of the rotating body, in which the rotating body has a to-be-irradiated portion that is an outer surface along an axis of the rotating body, the rotation sensor includes a light emitting element that emits an irradiation light beam toward the to-be-irradiated portion, and a light receiving element that receives a reflected light beam from the to-be-irradiated portion of the rotating body, the to-be-irradiated portion includes at least two regions having different intensities of the reflected light beam in a circumferential direction of the rotating body, and the intensity of the reflected light beam changes while the to-be-irradiated portion makes one rotation about the axis.

According to the present invention, an irradiation light beam is emitted toward the to-be-irradiated portion, which is the outer surface along the axis of the rotating body, and a reflected light beam from the to-be-irradiated portion is received, and then, the rotational state of the rotating body is detected based on a change in the current value corresponding to the intensity of the reflected light beam. Thus, the sizes of the medicinal solution administration device and the rotation detection device can be easily reduced.

### Brief Description of Drawings

FIG. 1 is a perspective view of a medicinal solution administration device according to an embodiment of the present invention.
FIG. 2 is a perspective view of a syringe pump unit.
FIG. 3 is a plan view of the syringe pump unit.
FIG. 4 is a perspective view of a second gear that is a rotating body.
FIG. 5A is a schematic view of a to-be-irradiated portion when a rotation angle is 0°. FIG. 5B is a schematic view of the to-be-irradiated portion when the rotation angle is 90°. FIG. 5C is a schematic view of the to-be-irradiated portion when the rotation angle is 180°. FIG. 5D is a schematic view of the to-be-irradiated portion when the rotation angle is 270°.
FIG. 6 is a graph illustrating a relationship between the rotation angle of the rotating body and the intensity of a reflected light beam.
FIG. 7 is a perspective view of a rotating body having a to-be-irradiated portion according to a first modification.
FIG. 8A is a schematic view of the to-be-irradiated portion according to the first modification when the rotation angle is 0°. FIG. 8B is a schematic view of the to-be-irradiated portion according to the first modification when the rotation angle is 90°. FIG. 8C is a schematic view of the to-be-irradiated portion according to the first modification when the rotation angle is 180°. FIG. 8D is a schematic view of the to-be-irradiated portion according to the first modification when the rotation angle is 270°.
FIG. 9 is a graph illustrating a relationship between the rotation angle of the rotating body having the to-be-irradiated portion according to the first modification and the intensity of a reflected light beam.
FIG. 10A is a schematic view of a to-be-irradiated portion according to a second modification when a rotation angle is 0°. FIG. 10B is a schematic view of the to-be-irradiated portion according to the second modification when the rotation angle is 90°. FIG. 10C is a schematic view of the to-be-irradiated portion according to the second modification when the rotation angle is 180°. FIG. 10D is a schematic view of the to-be-irradiated portion according to the second modification when the rotation angle is 270°.
FIG. 11 is a graph illustrating a relationship between the rotation angle of a rotating body having the to-be-irradiated portion according to the second modification and the intensity of a reflected light beam.

### Description of Embodiments

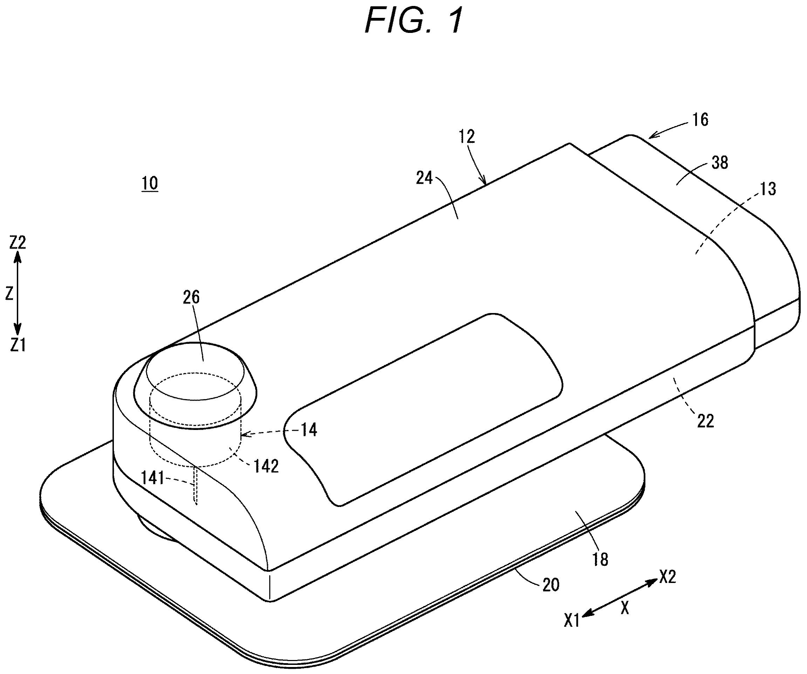

The medicinal solution administration device 10 illustrated in FIG. 1 is attached to the skin of a patient who is a living body, and is used for subcutaneous administration of a medicinal solution to the patient. The medicinal solution administration device 10 continuously administers a medicinal solution into a living body over a relatively long time (for example, about several minutes to several hours). The medicinal solution administration device 10 may intermittently administer the medicinal solution into the living body. Examples of the medicinal solution include protein preparations, such as insulin preparations, narcotic analgesics, and diuretics.

The medicinal solution administration device 10 includes a housing 12, a puncture portion 14, a syringe pump unit 16, an attachment member 18, and a release sheet 20.

The housing 12 extends in one direction (i.e., X direction). The housing 12 includes a bottom wall portion 22 forming a wall portion on one side in the thickness direction (i.e., Z direction) of the housing 12, and a ceiling portion 24 forming a wall portion on the other side in the thickness direction of the housing 12. An operation cover 26 is provided on the ceiling portion 24 at one end portion in the longitudinal direction (i.e., an end portion in the X1 direction) of the housing 12. When the operation cover 26 is operated (i.e., pressed), the puncture portion 14 is activated.

An insertion port 13 for inserting the syringe pump unit 16 into the housing 12 is provided at the other end portion in the longitudinal direction (i.e., an end portion in the X2 direction) of the housing 12.

The puncture portion 14 is attached to the housing 12. The puncture portion 14 has a needle member 142 including a puncture needle 141. The needle member 142 is movable in the puncture direction (i.e., Z1 direction) with respect to the housing 12. When the operation cover 26 is operated (i.e., pressed), the puncture needle 141 protrudes from the housing 12 in the Z1 direction due to a biasing force of a puncture biasing member (not illustrated), and punctures the skin.

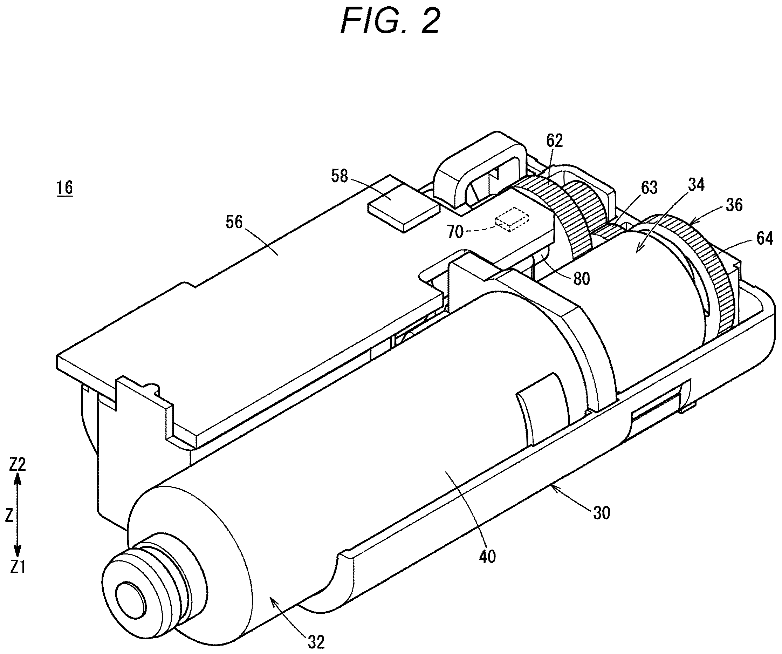

As illustrated in FIG. 2, the syringe pump unit 16 includes a chassis member 30, a prefilled syringe 32, a plunger 34, and a drive mechanism 36. FIG. 2 illustrates a state in which an upper cover 38 (see FIG. 1) of the syringe pump unit 16 is removed. The syringe pump unit 16 is arranged inside the housing 12 in a slidable manner along the longitudinal direction (i.e., X direction) of the housing 12.

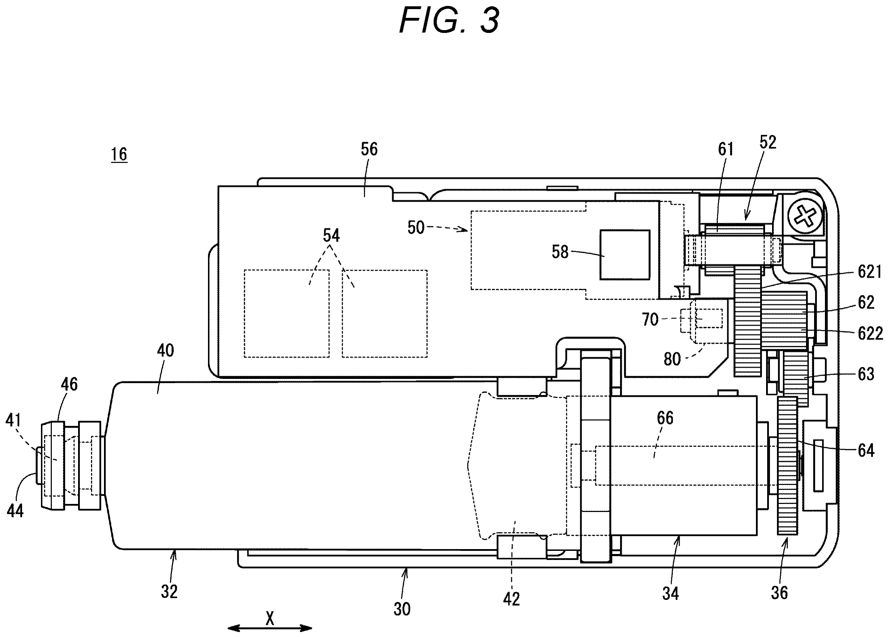

As illustrated in FIG. 3, the prefilled syringe 32 includes a medicinal solution container 40, a gasket 42, a sealing member 44, and a distal-end cap 46. The medicinal solution container 40 is supported by the chassis member 30. The medicinal solution container 40 has a medicinal solution discharge port 41 at its distal end. The inside of the medicinal solution container 40 is filled with a medicinal solution in advance.

The gasket 42 is formed of an elastic resin material, such as a rubber material or an elastomer material, and is slidably arranged inside the medicinal solution container 40. The sealing member 44 is formed of an elastic resin material, such as a rubber material or an elastomer material, and seals the medicinal solution discharge port 41 of the medicinal solution container 40 in a liquid-tight manner. The distal-end cap 46 fixes the sealing member 44 to the medicinal solution discharge port 41 of the medicinal solution container 40.

The plunger 34 pushes the gasket 42 in the medicinal solution container 40 toward its distal end to push out the medicinal solution in the medicinal solution container 40 through the medicinal solution discharge port 41. Although not illustrated in detail, the plunger 34 has an extendable structure. To this end, the plunger 34 has a plurality of tubular members overlapping in the radial direction. The plurality of tubular members are relatively displaceable in the axial direction (i.e., X direction) of the medicinal solution container 40. The plunger 34 can extend as the plurality of tubular members are relatively displaced in the axial direction. The plunger 34 pushes the gasket 42 toward the distal end and advances the gasket 42 by extending while moving toward the distal end.

The drive mechanism 36 includes a motor 50 and a power transmission unit 52. The motor 50 is supported by the chassis member 30. The power transmission unit 52 includes a plurality of rotating bodies. The plurality of rotating bodies include a first gear 61 coupled to an output shaft of the motor 50, a second gear 62 that meshes with the first gear 61, a third gear 63 that meshes with the second gear 62, and a fourth gear 64 that meshes with the third gear 63. The axes (i.e., rotation axes) of the first to fourth gears 61 to 64 are parallel to one another.

The first gear 61 is a pinion gear, and is arranged coaxially with the output shaft of the motor 50. The second gear 62 is an intermediate gear, and is rotationally driven by the first gear 61. The second gear 62 includes a large gear 621 that meshes with the first gear 61, and a small gear 622 that meshes with the third gear 63. The third gear 63 is an idle gear, and is rotationally driven by the second gear 62.

The fourth gear 64 is an output gear, and is rotationally driven by the third gear 63. The fourth gear 64 has a feed screw 66 fixed at its center. The feed screw 66 is threadably engaged with the plunger 34. Through the rotation of the feed screw 66, the plunger 34 moves toward the distal end while extending.

The power transmission unit 52 is not limited to the gear mechanism described in the present embodiment, and may include a belt mechanism having a belt and a plurality of pulleys, for example.

The syringe pump unit 16 further includes a battery 54, a circuit board 56, and a control unit 58. The battery 54 is held in a battery housing unit of the chassis member 30, and supplies power to the motor 50. The circuit board 56 is supported by the chassis member 30 at a position adjacent to the medicinal solution container 40. The control unit 58 is mounted on the circuit board 56, and includes a processor, such as a central processing unit (CPU), for example. The control unit 58 controls the rotation of the motor 50.

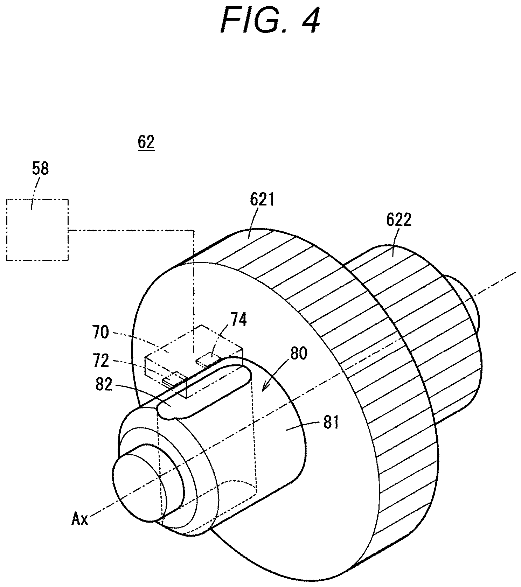

As illustrated in FIG. 2, the syringe pump unit 16 further includes a rotation sensor 70 that detects rotation of one of the plurality of rotating bodies. The rotation sensor 70 is arranged on a back surface (i.e., a surface facing the Z1 direction) of the circuit board 56, and faces a to-be-irradiated portion 80 described later. In the present embodiment, the rotation sensor 70 detects rotation of the second gear 62. As illustrated in FIG. 4, the rotation sensor 70 includes a light emitting element 72 that emits an irradiation light beam toward the to-be-irradiated portion 80, and a light receiving element 74 that receives a reflected light beam from the to-be-irradiated portion 80. The rotation sensor 70 is a reflective photoelectric sensor with a light receiving/emitting function.

The irradiation light beam emitted by the light emitting element 72 is an infrared light beam, for example. The irradiation light beam may also be a visible light beam. The light emitting element 72 may continuously emit an irradiation light beam, or may intermittently emit irradiation light beams at short intervals. If the light emitting element 72 intermittently emits irradiation light beams, the power consumption of the battery 54 can be reduced.

The configuration of the rotation sensor 70 is not limited to a configuration where it is formed as a single unit, like a reflective photoelectric sensor with a light receiving/emitting function, and may be a configuration where the light emitting element 72 and the light receiving element 74 are individually mounted on the circuit board 56.

The second gear 62, which is a rotating body, has the to-be-irradiated portion 80 that is an outer surface along the axis Ax (i.e., axis) of the second gear 62. The to-be-irradiated portion 80 is arranged on the axis Ax of the second gear 62. The to-be-irradiated portion 80 is a shaft portion of the second gear 62. The rotating body having the to-be-irradiated portion 80 and the above-described rotation sensor 70 form a rotation detection device. The to-be-irradiated portion 80 protrudes from the large gear 621 in the axial direction of the second gear 62. A cross-section of the to-be-irradiated portion 80 perpendicular to the axis Ax has a circular shape. That is, the to-be-irradiated portion 80 is formed in a cylindrical shape. The to-be-irradiated portion 80 has a diameter smaller than those of the large gear 621 and the small gear 622.

The to-be-irradiated portion 80 has at least two regions having different intensities of a reflected light beam in the circumferential direction of the second gear 62. In the present embodiment, the above-described at least two regions having different intensities of a reflected light beam are formed as a through-hole 82 penetrating the to-be-irradiated portion 80 in the radial direction of the circular shape is provided. The to-be-irradiated portion 80 is preferably formed of a material of a bright color (for example, a white-based color) to allow an outer peripheral surface 81 of the to-be-irradiated portion 80 to easily reflect light beams. Alternatively, the to-be-irradiated portion 80 may be formed of metal. A portion of the to-be-irradiated portion 80 on the inner side of its outer peripheral surface 81 may be formed of a material (for example, black resin) that hardly reflects light beams as long as the outer peripheral surface 81 is configured to easily reflect light beams (for example, formed of a reflective coating or a reflective sheet).

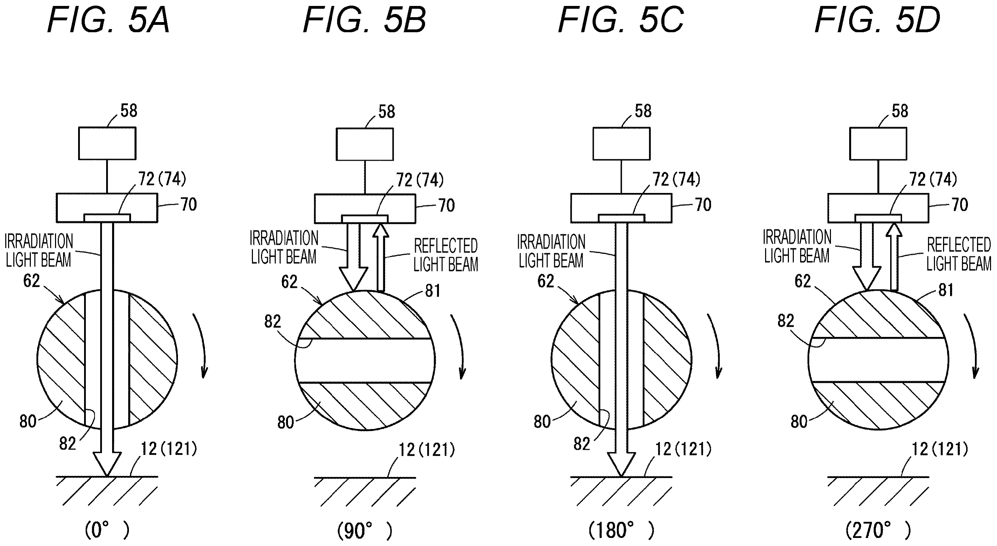

As illustrated in FIG. 5A, when an irradiation light beam passes through the through-hole 82, the irradiation light beam irradiates an inner wall (hereinafter referred to as a "light receiving wall 121") of the housing 12. The light receiving wall 121 is a wall that is arranged at a position facing the rotation sensor 70, and easily absorbs irradiation light beams. That is, the light receiving wall 121 is a wall that hardly reflects irradiation light beams. The light receiving wall 121 is a black wall, for example. The light receiving wall 121 may be a black sheet fixed to the inner wall of the housing 12. When an irradiation light beam passes through the through-hole 82, a reflected light beam is hardly generated, or even if generated, the amount of the reflected light beam generated is very small. Therefore, the intensity of the reflected light beam received by the light receiving element 74 of the rotation sensor 70 is low (i.e., relatively low).

As illustrated in FIG. 5B, when an irradiation light beam does not pass through through-hole 82 but irradiates the outer peripheral surface 81 of the to-be-irradiated portion 80, the irradiation light beam is reflected by the outer peripheral surface 81 of the to-be-irradiated portion 80, and the light receiving element 74 of the rotation sensor 70 receives a reflected light beam. Therefore, the intensity of the reflected light beam received by the light receiving element 74 of the rotation sensor 70 is high (i.e., relatively high). Hereinafter, a relatively low intensity of a reflected light beam will also be referred to as "dark". A relatively high intensity of a reflected light beam will also be referred to as "bright". In addition, the intensity level of a reflected light beam will also be referred to as "brightness".

The control unit 58 detects the rotational state of the second gear 62, which is a rotating body, based on a change in the current value corresponding to the intensity of a reflected light beam. Specifically, the control unit 58 measures the number of rotations of the second gear 62 based on a change in the current value corresponding to the intensity of a reflected light beam. Details of the operation of the control unit 58 will be described later.

In FIG. 1, the attachment member 18 is a flexible sheet-like member. The attachment member 18 is fixed to the bottom wall portion 22 of the housing 12. A surface of the attachment member 18 located on a side opposite to the housing 12 is provided with an attachment surface (i.e., adhesive surface) that can be attached to the skin of a living body. The release sheet 20 is releasably attached to the attachment surface of the attachment member 18 in the initial state of the medicinal solution administration device 10.

The medicinal solution administration device 10 is used as follows.

When the medicinal solution administration device 10 is used, a user inserts the syringe pump unit 16 into the housing 12. Accordingly, since a connection needle (not illustrated) provided inside the housing 12 penetrates the sealing member 44 (see FIG. 3) of the prefilled syringe 32, the medicinal solution in the prefilled syringe 32 can be sent to the puncture needle 141. Next, the user peels off the release sheet 20 from the attachment member 18 to expose the attachment surface of the attachment member 18. After peeling off the release sheet 20, the user attaches the attachment member 18 to the skin of the living body.

Next, when the user pushes the operation cover 26 in the Z1 direction, the needle member 142 including the puncture needle 141 is displaced in the Z1 direction (i.e., puncture direction) due to an elastic biasing force of the puncture biasing member (not illustrated). As a result, the puncture needle 141 punctures the skin of the living body. When the user turns on the medicinal solution administration device 10 after the puncturing of the skin with the puncture needle 141 is complete, the motor 50 illustrated in FIG. 3 is activated. The motor 50 advances the plunger 34 and the gasket 42 via the power transmission unit 52. Accordingly, the medicinal solution is administered to the patient.

With the medicinal solution administration device 10, it is possible to grasp the state of delivery of the medicinal solution based on the detection result of the rotation sensor 70. Specifically, the control unit 58 can grasp the state of delivery of the medicinal solution with the plunger 34 by measuring the number of rotations or the rotation speed of the second gear 62 based on the detection result of the rotation sensor 70. For example, items (1) to (4) below can be grasped by measuring the number of rotations or the rotation speed of the second gear 62.

(1) When the second gear 62 rotates at predetermined time intervals, the control unit 58 determines that the state of delivery of the solution is normal.
(2) When the number of rotations of the second gear 62 has reached a predetermined number, the control unit 58 determines that the delivery of the solution is complete.
(3) When the rotation speed of the second gear 62 has become slower than the normal speed in a state where the medicinal solution remains in the medicinal solution container 40, the control unit 58 determines that a medicinal solution flow path from the medicinal solution discharge port 41 of the medicinal solution container 40 to the needle tip of the puncture needle 141 is about to be blocked.
(4) When the rotation of the second gear 62 has stopped in a state where the medicinal solution remains in the medicinal solution container 40, the control unit 58 determines that the medicinal solution flow path is blocked.

As described above, the to-be-irradiated portion 80 has at least two regions having different intensities of a reflected light beam in the circumferential direction of the second gear 62. Therefore, the intensity of a reflected light beam changes while the to-be-irradiated portion 80 makes one rotation about the axis Ax. The control unit 58 detects the rotational state of the second gear 62 based on a change in the current value corresponding to the intensity of the reflected light beam.

In the present embodiment, as illustrated in FIGS. 5A to 5D, since the to-be-irradiated portion 80 is provided with the through-hole 82, the intensity level of a reflected light beam (i.e., the brightness of a reflected light beam) received by the light receiving element 74 alternately changes with the rotation of the second gear 62. Specifically, as illustrated in FIGS. 5A and 5C, when the rotation angle of the second gear 62 is 0° and 180°, an irradiation light beam passes through the through-hole 82, so that the intensity of a reflected light beam is low (i.e., dark). As illustrated in FIGS. 5B and 5D, when the rotation angle of the second gear 62 is 90° and 270°, an irradiation light beam is reflected by the outer peripheral surface 81 of the to-be-irradiated portion 80, so that the intensity of a reflected light beam is high (i.e., bright).

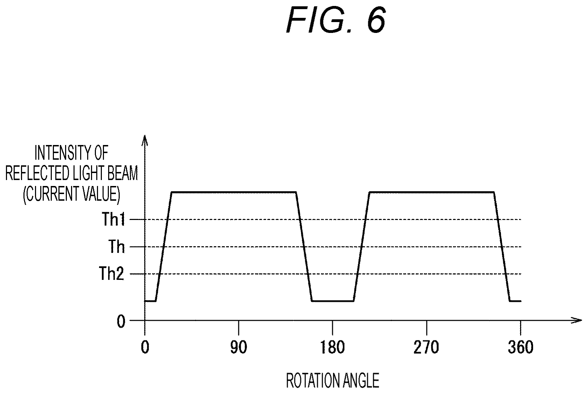

Therefore, the intensity of a reflected light beam changes as illustrated in FIG. 6. Since the light receiving element 74 generates an electric signal (i.e., current) whose magnitude corresponds to the intensity of the reflected light beam, the current value also changes with the rotation of the second gear 62. A change in the current value from a low level to a high level and a change in the current value from a high level to a low level indicate that the second gear 62 is rotating.

As illustrated in FIG. 6, in the present embodiment, while the second gear 62 makes one rotation, the current value changes from a low level to a high level twice, and the current value changes from a high level to a low level twice (there are four changes in total). Therefore, the control unit 58 can detect the rotational state of the second gear 62 based on a change in the current value corresponding to the intensity of a reflected light beam. Examples of the rotational state of the second gear 62 detected by the control unit 58 include the number of rotations and the rotation speed of the second gear 62. The control unit 58 may detect only one of the number of rotations and the rotation speed of the second gear 62.

The manner of determining "a change in the current value" described above is not limited to a particular manner. For example, a predetermined threshold Th is set at an intermediate level between a high current value and a low current value. In such a case, the control unit 58 determines a change in the current value from a range exceeding the threshold Th to a range of less than or equal to the threshold Th and a change in the current value from a range of less than or equal to the threshold Th to a range exceeding the threshold Th as a "change in the current value", and thus detects the rotation of the second gear 62.

Alternatively, two thresholds (i.e., a first threshold Th1 and a second threshold Th2) that are high and low may be set for the current value. In such a case, the control unit 58 determines a current value that exceeds the first threshold Th1 as a high current value, determines a current value that falls below the second threshold Th2 as a low current value, and determines a change in the current value from a high current value to a low current value and a change in the current value from a low current value to a high current value as a "change in the current value", and thus detects the rotation of the second gear 62.

The present embodiment achieves the following advantageous effects.

As illustrated in FIGS. 5A to 5D, the rotation sensor 70 emits an irradiation light beam toward the to-be-irradiated portion 80, which is an outer surface along the axis Ax of the rotating body (i.e., the second gear 62), and receives a reflected light beam from the to-be-irradiated portion 80. The control unit 58 (see FIG. 3) detects the rotational state of the rotating body based on a change in the current value corresponding to the intensity of the reflected light beam. Therefore, it is easy to reduce the size of the medicinal solution administration device 10 illustrated in FIG. 1 as compared with a configuration in which rotation is detected using an encoder that protrudes significantly from a motor shaft in the radial direction.

As illustrated in FIG. 4, a cross-section of the to-be-irradiated portion 80 perpendicular to the axis Ax has a circular shape, and the to-be-irradiated portion 80 is provided with the through-hole 82 penetrating therethrough in the radial direction of the circular shape. According to such a configuration, at least two regions having different intensities of a reflected light beam can be easily provided.

The control unit 58 measures the number of rotations of the rotating body (i.e., the second gear 62) based on a change in the current value corresponding to the intensity of the reflected light beam. Measuring the number of rotations makes it possible to grasp whether the delivery of the medicinal solution is complete.

The rotating body provided with the to-be-irradiated portion 80 is not limited to the second gear 62. As another first aspect, the first gear 61, the third gear 63, or the fourth gear 64 illustrated in FIG. 3 may be provided with the to-be-irradiated portion 80. When the power transmission unit 52 includes a belt mechanism instead of the gear mechanism, any one of pulleys may be provided with the to-be-irradiated portion 80, as another second aspect. The other first and second aspects also apply to first and second modifications described later.

Instead of the to-be-irradiated portion 80 having the through-hole 82 illustrated in FIG. 4 and the like, the first modification or the second modification described below may be adopted.

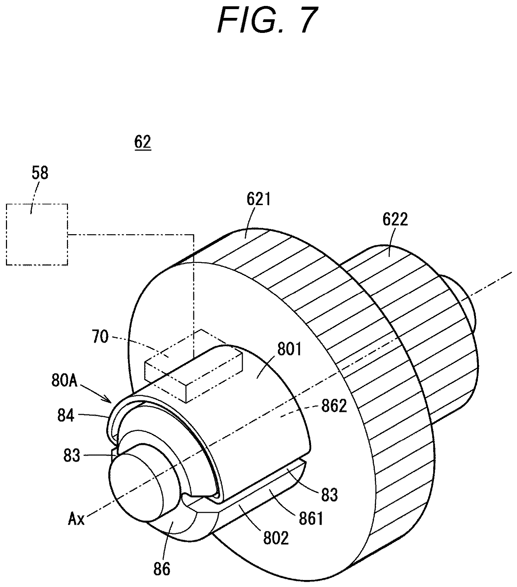

A to-be-irradiated portion 80A according to the first modification illustrated in FIG. 7 includes a metal portion 84 and a resin portion 86 having an intensity of a reflected light beam lower than that of the metal portion 84. A cross-section of the to-be-irradiated portion 80A, which includes the metal portion 84 and the resin portion 86, perpendicular to the axis Ax has a circular shape. That is, the to-be-irradiated portion 80A has a substantially cylindrical shape.

The resin portion 86 is formed of a resin material colored in black, for example, to lower the intensity of a reflected light beam from the resin portion 86. The resin portion 86 has a pair of grooves 83 formed therein along the axial direction of the second gear 62 at positions opposite to each other in the circumferential direction.

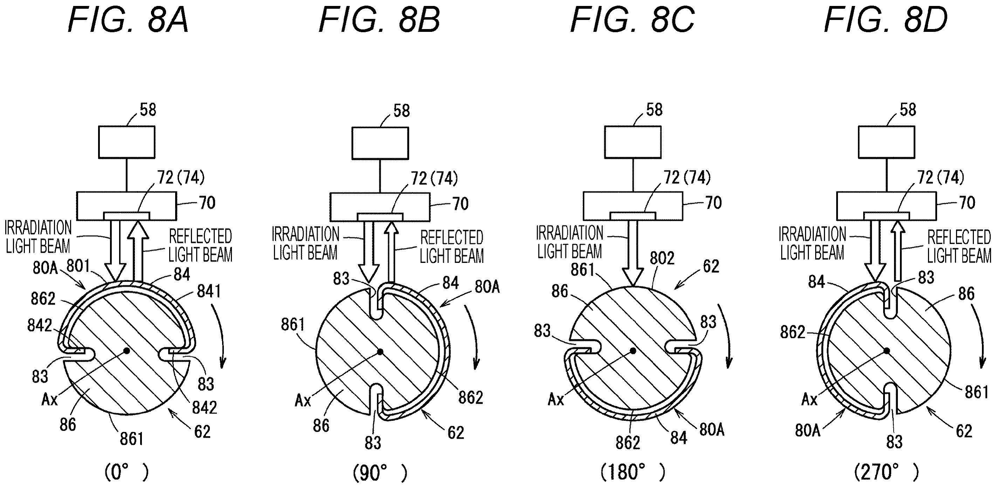

As illustrated in FIG. 8A, a portion of the outer peripheral surface of the resin portion 86 that is not covered with the metal portion 84 (hereinafter referred to as an "exposed outer peripheral surface 861") is an arcuate surface. A portion of the outer peripheral surface of the resin portion 86 that is covered with the metal portion 84 (hereinafter referred to as a "covered outer peripheral surface 862") is an arcuate surface. The radius of curvature of the covered outer peripheral surface 862 is smaller than that of the exposed outer peripheral surface 861.

Examples of a metal material forming the metal portion 84 include stainless steel and an aluminum alloy. The metal portion 84 favorably reflects an irradiation light beam emitted by the rotation sensor 70. The metal portion 84 has an arc portion 841 along the outer peripheral surface of the resin portion 86, and a pair of protrusions 842 protruding from opposite ends of the arc portion 841 toward the inside of the to-be-irradiated portion 80A. The arc portion 841 covers the covered outer peripheral surface 862 of the resin portion 86.

The radius of curvature of the outer surface of the arc portion 841 is substantially the same as that of the exposed outer peripheral surface 861 of the resin portion 86. The radius of curvature of the outer surface of the arc portion 841 may be different from that of the exposed outer peripheral surface 861 of the resin portion 86. The pair of protrusions 842 is inserted into and engaged with the pair of grooves 83 of the resin portion 86.

In FIG. 7, the to-be-irradiated portion 80A has a first to-be-irradiated surface 801 and a second to-be-irradiated surface 802 having an intensity of a reflected light beam lower than that of the first to-be-irradiated surface 801. The outer surface of the arc portion 841 of the metal portion 84 corresponds to the first to-be-irradiated surface 801. The exposed outer peripheral surface 861 of the resin portion 86 corresponds to the second to-be-irradiated surface 802. Instead of the metal portion 84, a reflective coating or a reflective sheet that easily reflects light beams may be fixed to the covered outer peripheral surface 862 of the resin portion 86. In such a case, the reflective coating or the reflective sheet corresponds to the first to-be-irradiated surface 801.

The lengths of the arc portion 841 and the exposed outer peripheral surface 861 of the resin portion 86 in the circumferential direction of the to-be-irradiated portion 80A are substantially the same. Therefore, substantially half of the outer peripheral surface of the to-be-irradiated portion 80A corresponds to the arc portion 841, and the other half corresponds to the exposed outer peripheral surface 861. In other words, half of the circumference of the outer surface of the to-be-irradiated portion 80A corresponds to the first to-be-irradiated surface 801, and the other half of the circumference of the outer surface of the to-be-irradiated portion 80A corresponds to the second to-be-irradiated surface 802. Therefore, the angle over which each of the first to-be-irradiated surface 801 and the second to-be-irradiated surface 802 extends in the circumferential direction of the to-be-irradiated portion 80A is about 180°. Note that the angle over which the first to-be-irradiated surface 801 extends in the circumferential direction of the to-be-irradiated portion 80A is not limited to about 180°. The angle over which the first to-be-irradiated surface 801 extends may be set in the range of 160° to 200°, for example.

As illustrated in FIG. 8A, when the rotation angle of the second gear 62 is 0°, an irradiation light beam is reflected by the outer peripheral surface of the metal portion 84 (i.e., the first to-be-irradiated surface 801), so that the intensity of a reflected light beam is high (i.e., bright). As illustrated in FIG. 8C, when the rotation angle of the second gear 62 is 180°, an irradiation light beam irradiates the resin portion 86 (i.e., the second to-be-irradiated surface 802), so that the intensity of a reflected light beam is low (i.e., dark). When the rotation angle of the second gear 62 is 90° as illustrated in FIG. 8B and when the rotation angle of the second gear 62 is 270° as illustrated in FIG. 8D, the intensity of a reflected light beam is at an intermediate level between the high level (FIG. 8A) and the low level (FIG. 8C).

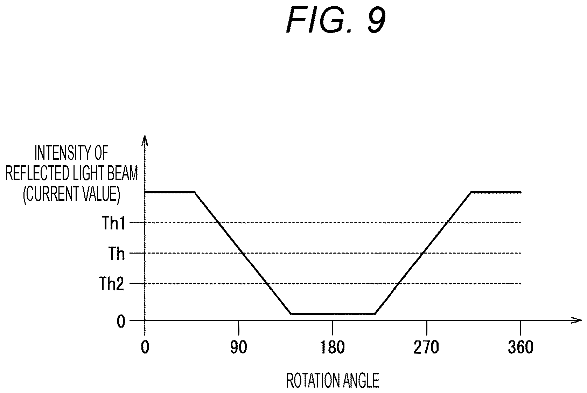

FIG. 9 illustrates a change in the intensity of a reflected light beam (i.e., a change in the current value) while the second gear 62 having the to-be-irradiated portion 80A makes one rotation. The current value changes from a high level to a low level once in the range of 0° to 180°, and the current value changes from a low level to a high level once in the range of 180° to 360° (there are two changes in total). Therefore, the control unit 58 can detect the rotational state of the second gear 62 based on a change in the current value corresponding to the intensity of a reflected light beam.

As described above, in the first modification, the number of times that the control unit 58 detects a change in the intensity of a reflected light beam (i.e., a change in the current value) while the second gear 62 makes one rotation is two. Accordingly, it is possible to achieve a detection resolution in which one detection of a change in the intensity of a reflected light beam corresponds to the forward movement of the plunger 34 by about 0.1 mm. A design that serves as a premise for the foregoing will be described below with reference to FIG. 3.

A screw pitch of the feed screw 66 is 0.5 mm. The plunger 34 moves forward by 0.5 mm while the fourth gear 64 makes one rotation. When the plunger 34 moves forward by 0.5 mm, the second gear 62 provided with the to-be-irradiated portion 80A makes about 2.5 rotations. If a change in the intensity of a reflected light beam can be detected twice while the second gear 62 makes one rotation, it is possible to detect a change in the intensity of a reflected light beam five times each time the plunger 34 moves forward by about 0.5 mm. Therefore, it is possible to detect a change in the intensity of a reflected light beam once each time the plunger 34 moves forward by about 0.1 mm. Note that the above design is only an example, and the present invention is not limited to the numerical values of the above design.

When the to-be-irradiated portion 80A according to the first modification illustrated in FIG. 7 is adopted, the following advantageous effects are obtained.

The outer peripheral surface of the to-be-irradiated portion 80A includes the first to-be-irradiated surface 801 and the second to-be-irradiated surface 802 having an intensity of a reflected light beam lower than that of the first to-be-irradiated surface 801. According to such a configuration, at least two regions having different intensities of a reflected light beam can be easily provided.

As compared with the to-be-irradiated portion 80 having the through-hole 82 illustrated in FIG. 4, in the to-be-irradiated portion 80A illustrated in FIG. 7, there is no influence of reflection on the inner surface of the through-hole 82, so that a difference in the intensity of a reflected light beam due to a difference in the rotation angle can be increased. For example, the maximum value of the current value while the to-be-irradiated portion 80A makes one rotation about the axis Ax is 10 times or more the minimum value of the current value. Setting a current ratio corresponding to the level (i.e., brightness) of a reflected light beam to 10 times or greater can improve the accuracy of rotation detection. To further increase the current ratio, plating may be applied to the outer surface of the metal portion 84 to form a surface that reflects light beams more easily.

The to-be-irradiated portion 80A includes the metal portion 84 and the resin portion 86 having an intensity of a reflected light beam lower than that of the metal portion 84. The metal portion 84 is provided with the first to-be-irradiated surface 801, and the resin portion 86 is provided with the second to-be-irradiated surface 802. According to such a configuration, two regions having different intensities of a reflected light beam can be easily provided in the to-be-irradiated portion 80A.

A cross-section of the to-be-irradiated portion 80A perpendicular to the axis Ax has a circular shape. Half of the circumference of the outer surface of the to-be-irradiated portion 80A corresponds to the first to-be-irradiated surface 801, and the other half of the circumference of the outer surface of the to-be-irradiated portion 80A corresponds to the second to-be-irradiated surface 802. According to such a configuration, a region where the intensity of a reflected light beam is high and a region where the intensity of a reflected light beam is low while the to-be-irradiated portion 80A makes one rotation are made substantially equal in size. Thus, the resolution of rotation detection can be enhanced.

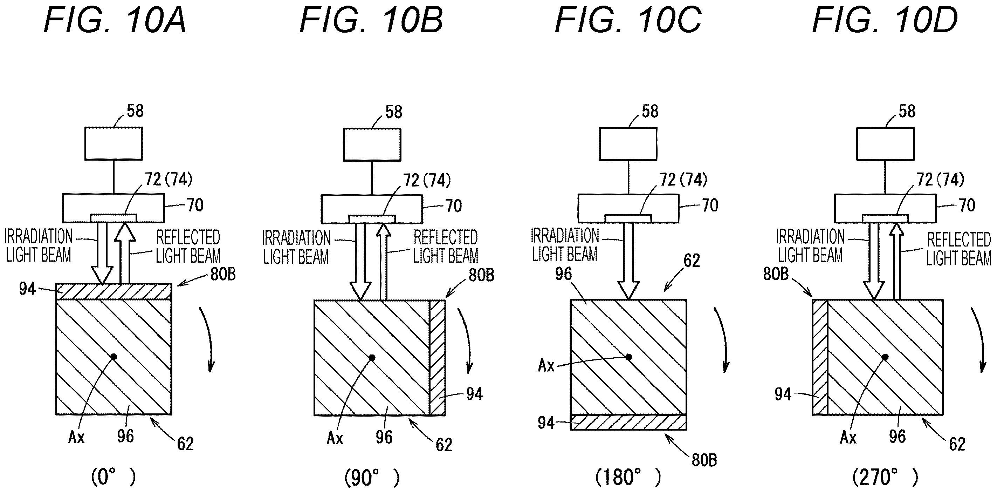

A to-be-irradiated portion 80B according to the second modification illustrated in FIGS. 10A to 10D includes a metal portion 94 and a resin portion 96 having an intensity of a reflected light beam lower than that of the metal portion 94. The to-be-irradiated portion 80B according to the second modification corresponds to an aspect obtained by changing the shape of the to-be-irradiated portion 80A according to the first modification. A cross-section of the to-be-irradiated portion 80B perpendicular to the axis Ax of the to-be-irradiated portion 80B has a polygonal shape. Since the to-be-irradiated portion 80B illustrated as an example in the drawing has a quadrangular shape, the metal portion 94 and the resin portion 96 will be described below assuming that the to-be-irradiated portion 80B has a quadrangular shape.

The metal portion 94 is formed of a flat metal plate. The resin portion 96 is formed in the shape of a quadrangular prism. The resin portion 96 is formed of a resin material colored in black, for example, to lower the intensity of a reflected light beam from the resin portion 96. The metal portion 94 is fixed to one side of the quadrangular prism-shaped resin portion 96. Instead of the metal portion 94, a reflective coating or a reflective sheet that easily reflects light beams may be fixed to the resin portion 96.

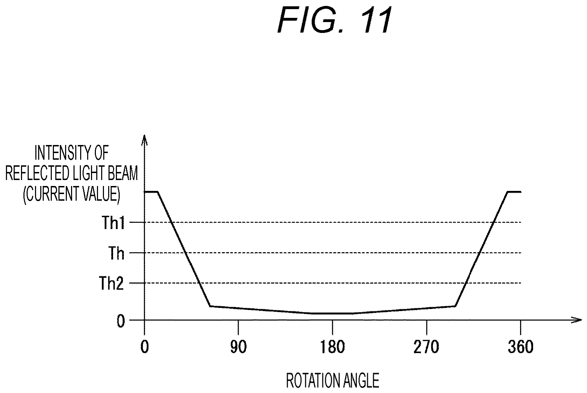

FIG. 11 illustrates a change in the intensity of a reflected light beam (i.e., a change in the current value) while the second gear 62 having the to-be-irradiated portion 80B makes one rotation. The current value changes from a high level to a low level once in the range of 0° and 90°, and the current value changes from a low level to a high level once in the range of 270° and 360°. Therefore, the control unit 58 can detect the rotational state of the second gear 62 based on a change in the current value corresponding to the intensity of a reflected light beam.

As described above, even when the to-be-irradiated portion 80B having a polygonal cross-section is adopted, it is possible to detect the rotational state of the second gear 62, which is a rotating body, based on a change in the current value corresponding to the intensity of a reflected light beam.

Note that a metal plate may be fixed to each of two of the four sides of the quadrangular prism-shaped resin portion 96. In such a case, the two metal plates may be arranged in an L-shape on two adjacent sides of the resin portion 96, or may be arranged on two opposite sides of the resin portion 96.

Note that the present invention is not limited to the above-described disclosure, and various configurations can be adopted without departing from the gist of the present invention.

## Claims

1. A medicinal solution administration device
comprising:
a medicinal solution container that stores a medicinal solution, and has a medicinal solution discharge port at a distal end;
a plunger that pushes out the medicinal solution in the medicinal solution container through the medicinal solution discharge port;
a drive mechanism including a motor and a rotating body that transmits rotation of the motor to the plunger, the drive mechanism being adapted to move the plunger toward the distal end;
a rotation sensor for detecting rotation of the rotating body; and
a control unit, wherein
the rotating body has a to-be-irradiated portion that is an outer surface along an axis of the rotating body,
the rotation sensor includes a light emitting element that emits an irradiation light beam toward the to-be-irradiated portion, and a light receiving element that receives a reflected light beam from the to-be-irradiated portion of the rotating body,
the to-be-irradiated portion includes at least two regions having different intensities of the reflected light beam in a circumferential direction of the rotating body,
the intensity of the reflected light beam changes while the to-be-irradiated portion makes one rotation around the axis, and
the control unit detects a rotational state of the rotating body based on a change in a current value corresponding to the intensity of the reflected light beam.

2. The medicinal solution administration device according to claim 1, wherein
an outer peripheral surface of the to-be-irradiated portion includes a first to-be-irradiated surface and a second to-be-irradiated surface having an intensity of the reflected light beam lower than that of the first to-be-irradiated surface.

3. The medicinal solution administration device according to claim 2, wherein
the to-be-irradiated portion includes a metal portion and a resin portion having an intensity of the reflected light beam lower than that of the metal portion, and
the metal portion is provided with the first to-be-irradiated surface, and the resin portion is provided with the second to-be-irradiated surface.

4. The medicinal solution administration device according to claim 2, wherein
a cross-section of the to-be-irradiated portion perpendicular to the axis has a circular shape,
half of a circumference of an outer surface of the to-be-irradiated portion corresponds to the first to-be-irradiated surface, and another half of the circumference of the outer surface of the to-be-irradiated portion corresponds to the second to-be-irradiated surface.

5. The medicinal solution administration device according to claim 1, wherein
a cross-section of the to-be-irradiated portion perpendicular to the axis has a circular shape, and
the to-be-irradiated portion is provided with a through-hole penetrating the to-be-irradiated portion in a radial direction of the circular shape.

6. The medicinal solution administration device according to claim 1, wherein
a cross-section of the to-be-irradiated portion perpendicular to the axis has a polygonal shape.

7. The medicinal solution administration device according to any one of claims 1 to 6, wherein
a maximum value of the current value while the to-be-irradiated portion makes one rotation about the axis is 10 times or more a minimum value of the current value.

8. The medicinal solution administration device according to any one of claims 1 to 6, wherein
the control unit measures the number of rotations of the rotating body based on a change in the current value corresponding to the intensity of the reflected light beam.

9. A rotation detection device comprising:
a rotating body; and
a rotation sensor that detects rotation of the rotating body, wherein
the rotating body has a to-be-irradiated portion that is an outer surface along an axis of the rotating body,
the rotation sensor includes a light emitting element that emits an irradiation light beam toward the to-be-irradiated portion, and a light receiving element that receives a reflected light beam from the to-be-irradiated portion of the rotating body,
the to-be-irradiated portion includes at least two regions having different intensities of the reflected light beam in a circumferential direction of the rotating body, and
the intensity of the reflected light beam changes while the to-be-irradiated portion makes one rotation about the axis.
